# EUROPEAN PATENT APPLICATION

(11) **EP 4 417 974 A1**
(43) Date of publication of application: **21.08.2024**
(21) Application number: 23382150.3
(22) Date of filing: 17.02.2023
(51) Int. Cl.: G01N 33/569, A61K 39/00

(54) **CD33 AS A BIOMARKER OF HIV CONTROL**

(71) Applicant: Fundació Privada Institut de Recerca de la SIDA-Caixa, 08916 Badalona (ES); Institució Catalana de Recerca I Estudis Avançats, 08010 Barcelona (ES)
(72) Inventor: RUIZ RIOL, Marta, 08916 Badalona (Barcelona) (ES); DURAN CASTELLS, Clara, 08916 Badalona (Barcelona) (ES); BRADER-SILVA, Christian, 08010 Barcelona (ES)
(74) Representative: Elzaburu S.L.P.

(57) **Abstract**

The present invention relates to the use of CD33 as a biomarker of the response to a T-cell vaccine in a HIV-1 infected subject and to a method for the prognosis of the response of a HIV-1 infected subject to a T-cell vaccine comprising analyzing the CD33 levels in a sample from said subject, as well as of the use of an inhibitor or CD33 for the treatment and/or prevention of HIV-1 infection.

## Description

### FIELD OF THE INVENTION

The present invention relates to the use of CD33 as a biomarker of the response to a T-cell vaccine in a HIV-1 infected subject and to a method for the prognosis of the response of a HIV-1 infected subject to a therapy against HIV-1, by analyzing the CD33 levels in a sample from said subject. Also, the present invention relates to a CD33 inhibitor for use in the treatment and/or prevention of HIV-1 infection.

### BACKGROUND

Since its identification in the 1980*'*s, the human immunodeficiency virus HIV type 1 represents a major global public health issue. Although combined antiretroviral therapy (cART) effectively suppresses HIV viral replication and prevents accelerated disease progression, no effective cure for HIV infection exists. Moreover, cART is associated with viral resistance and treatment-related side effects and, even more important, is not accessible worldwide, reinforcing the urgent need for a lasting HIV cure. During the last decade, different cure strategies have been under development, some of them using so-called latency-reversing agents (LRA) such as histone deacetylase inhibitors that can open the chromatin and thereby enabling virus transcription. However, their use can also impact host gene expression (1), which in the case of the LRA romidepsin, has been associated with beneficial effects on the central nervous system regulating synaptic plasticity.

BCN02 was a single-arm, open-label clinical trial (NCT02616874) that enrolled 15 early-treated HIV infected individuals to receive a combination of the latency-reversing agent romidepsin (RMD) with MVA.HIVconsv vaccination (2, 3). The MVA.HIVconsv immunogen is formed by a chimeric protein assembled from 14 highly conserved domains derived from HIV genes Gag, Pol, Vif, and Env (2)(3). In the other hand, RMD has shown increased histone-3 acetylation levels causing a relaxation of the chromatin and consequently reactivation of latent HIV virus in ex vivo experiments (4). Importantly, the BCN02 clinical trial also included a monitored antiretroviral pause (MAP) for a period of up to 32 weeks to evaluate the clinical efficacy of the intervention. Of 13 individuals evaluated during MAP, 10 individuals reached ART-restart criteria (MAP non controllers, MAP-NC) during MAP while 3 participants were able to control the virus during the entire 32 weeks MAP period to levels below 2,000 HIV copies/ml (2).

The outcome in BCN02 has been linked to specific signatures in the host microbiome and the host gene epigenetic landscape (5, 6).

CD33 (Siglec-3) is a transmembrane receptor protein expressed by cells of myeloid lineage. It binds sialic acids, therefore is a member of the SIGLEC family of lectins. CD33 can modulate immune responses partly due to its ability to inhibit monocytes (12) but can also be expressed in dendritic cells and to a lesser extent on activated T cells and natural killer cells. The binding with its ligands, C1q and sialylated glycoproteins, leads to the recruitment of inhibitory proteins via its immunoreceptor tyrosine-based inhibition motif domains, which initiates signaling cascades that inhibit cell activation and functions associated with cytokine release or virus phagocytosis (13). Homeostatic basal levels of CD33 are low but are strongly increased under pathogenic conditions, including viral infections, HIV-1 included (13).

At present, there is as much a need for biomarkers to predict the response to therapeutic T-cell vaccination in a HIV-1 infected subject, as there is for specific biomarkers that related to control of infection and disease progression. There is also an urgent need for novel therapeutic interventions that can block viral replication, while avoiding side effects associated with current antiretroviral treatment modalities.

### DESCRIPTION OF THE INVENTION

The inventors have surprisingly found CD33 as a specific and significant biomarker that can discriminate MAP-C from MAP-NC and is additionally associated with HIV proviral levels, plasma viral loads, and neurological damage. Thus, analyzing the levels of CD33 in a sample from a HIV-1 infected subject is useful for predicting the response of said subject to a T-cell vaccine, as well as their viral load and viral reservoir size, both factors that impact outcome to therapeutic interventions (Golnaz Namazi et al. The Journal of Infectious Diseases 2018;218:1954-63; Bailón L et al. Nat Med 2022 Dec;28(12):2611-2621).

In a first aspect, the present invention relates to the use of CD33 as a biomarker of the response to a T-cell vaccine in a HIV-1 infected subject.

The term "response" as used herein, relates to the immune response in the vaccinated subject, including the clinical effect of the vaccination on its immune response (i.e.: vaccine specific T cell responses) and also including the degree of control of viral replication, considering that the viral replication is controlled when the subject has below 2,000 HIV-1 RNA copies/ml in plasma. Preferably, the term "response" as used herein relates to the degree of control of viral replication. In a preferred embodiment of the first aspect, a positive response to the vaccine is delaying the time until viral rebound and/or controlling the plasma virus under 2,000 HIV-1 RNA copies/ml, preferably under 1,000 HIV-1 RNA copies/ml, more preferably under 500 HIV-1 RNA copies/ml, even more preferably under 50 HIV-1 RNA copies/ml, upon the interruption of the combined antiretroviral therapy. As used herein, the term "rebound" relates to the viral load in the plasma of the subject being detectable, i.e. being above 50 HIV-1 RNA copies/ml.

In a preferred embodiment of the first aspect, the T-cell vaccine is MVA.HIVconsv vaccine or a HTI immunogen-based vaccine. MVA.HIVconsv vaccine is disclosed in Hanke, T., and McMichael, A. J. (2000). Nat. Med 6 (9), 951-955. HTI immunogen-based vaccines are disclosed in WO 2013/110818 and US9,988,425. In a preferred embodiment, from 1 to 4 doses (preferably 2 or 3 doses) of the vaccine are administered with 2 weeks and 6 months between doses, preferably with 1 to 6 months between doses, more preferably with 8 to 16 weeks between doses. Each dose of the vaccine comprises preferably between 10⁸ and 5×10⁸ plaque forming units of vector (pfu).

In a preferred embodiment of the first aspect, the levels of CD33 mRNA or CD33 protein are used as biomarker, preferably the levels of CD33 protein are used as biomarker, more preferably the levels of soluble CD33 protein are used as biomarker. In a preferred embodiment, the levels of soluble CD33 protein in the plasma are used as biomarker. The skilled person is aware of the many techniques which are available to analyze and quantify the levels of CD33 mRNA or protein, as well as the many commercially available tools to do so. For example, RT-PCR can be used for analyzing the levels of mRNA, and immune techniques based on antibodies against CD33 may be used for analyzing the levels of protein.

In a preferred embodiment of the first aspect, said use is performed in a sample from said subject, where the sample is selected from blood, plasma, serum, peripheral blood mononuclear cells (PBMCs), urine, cerebrospinal fluid and combinations thereof, preferably the sample is selected from blood, plasma, serum, PBMCs and combinations thereof, more preferably the sample is plasma. When CD33 mRNA levels are quantified, the sample is preferably full blood or PBMCs, more preferably, PBMCs.

In a preferred embodiment of the first aspect, the subject is human. In a preferred embodiment of the first aspect, the subject is under a therapy to control viral load and/or virus replication. Preferably, said therapy is combined antiretroviral therapy (cART) or anti CD33 therapy. In an embodiment, the subject is under therapy (cART or anti CD33) and interrupts said therapy after receiving the vaccine. The skilled person is fully aware that cART refers to the combinations of drugs that are used to keep HIV infections under control, consisting of a minimum of two drugs from two different drug classes that work by blocking various stages of the virus's replication cycle. At present, there are more than 25 individual antiretroviral drugs approved by the Food and Drug Administration (FDA), as well as more than 20 fixed-dose combination drugs comprised of two or more antiretroviral agents. Anti CD33 therapy is understood as any therapy directed to inhibit CD33 that reduces the HIV-1 viral load and/or the HIV-1 proviral levels and/or HIV-1 viral replication. Anti CD33 therapy is therefore the use of inhibitors of CD33 that reduce viral replication and integration. Examples of anti CD33 therapy are anti CD33 antibodies, siRNAs, CD33 inhibitors or fragments thereof.

In a preferred embodiment of the first aspect, the CD33 levels are analyzed:
a. before administering the vaccine,
b. after administering the vaccine,
c. before interrupting the therapy to control viral load and/or replication,
d. after interrupting the therapy to control viral load and/or replication, or
e. any combination of (a) and (c), or (b) and (c) or (b) and (d).

In a preferred embodiment of the first aspect, further to the vaccine, the subject is also administered a latency reversing agent. Preferably, the latency reversing agent is a histone deacetylase inhibitor, more preferably, the latency reversing agent is romidepsin. A latency reversing agent (LRA), as used herein, is any drug used to increase viral gene transcription so that latently infected cells start producing virus and become visible to the immune system. Classes of LRAs and examples of LRAs are well known by the skilled person, as shown i.e.: in Kim Y, et al. Cell Host and Microbe, 2018; 23, 1, 14-26. For example, when the LRA is the histone deacetylase inhibitor romidepsin, it is administered in three weekly doses of 5 mg/m².

The inventors have surprisingly found that untreated HIV-infected subjects show a positive relationship between CD33 plasma and PBMC gene expression levels and viral parameters. Thus, a second aspect of the present invention relates to the use of CD33 as a biomarker of viral load, viral reservoir and/or viral replication in a HIV-1 infected subject. In a preferred embodiment of the second aspect, the levels of CD33 mRNA or CD33 protein are used as biomarker, preferably the CD33 protein plasma levels or the CD33 mRNA levels in PBMCs.

Also, the inventors have surprisingly found that plasma levels of CD33 were also significantly correlated with markers of neurological damage. Therefore, another aspect of the present invention relates to the use of CD33 as a biomarker of neurological damage in a HIV-1 infected subject. In a preferred embodiment of this aspect, CD33 is used as a biomarker of the response to a T-cell vaccine and of neurological damage in a HIV-1 infected subject.

As a natural consequence of the first aspect, the present invention relates also to a method for the prognosis of the response of a HIV-1 infected subject to a T-cell vaccine comprising:
a. analyzing the CD33 levels in a sample from said subject;
b. administering the vaccine to the subject; and
c₁. prognosing that the viral replication will be controlled, if the results of step (a) correspond to the low levels found in the group of subjects that respond to the vaccine; or
c₂. prognosing that the viral replication will not be controlled, if the results of step (a) correspond to the high levels found in the group of subjects that do not respond to the vaccine.

In a preferred embodiment of this aspect, the levels of CD33 mRNA or CD33 protein are analyzed, preferably the levels of CD33 protein are analyzed, more preferably the levels of soluble CD33 protein are analyzed. In a preferred embodiment, the levels of soluble CD33 protein in the plasma are analyzed. In a preferred embodiment of this aspect, the sample is selected from blood, plasma, serum, peripheral blood mononuclear cells (PBMCs), urine, cerebrospinal fluid and combinations thereof, preferably the sample is selected from blood, plasma, serum, PBMCs and combinations thereof, more preferably the sample is plasma. When CD33 mRNA levels are quantified, the sample is preferably full blood or PBMCs, more preferably, PBMCs.

In a preferred embodiment, the subject is under a therapy to control viral load before the vaccination, and interrupts said therapy after vaccination. The response to the vaccination is understood as positive when viral replication is controlled upon the interruption of said therapy. In a preferred embodiment of this aspect, after step c₂, the subject restarts the therapy to control viral load. In a preferred aspect, the therapy is antiretroviral therapy or anti-CD33 therapy. In a preferred embodiment, the therapy is combined antiretroviral therapy.

In a preferred embodiment of this aspect, the T-cell vaccine is MVA.HIVconsv vaccine or a HTI immunogen based vaccine. Preferred embodiments in relation to the vaccines are those of the first aspect.

In a preferred embodiment of this aspect, step (b) further comprises administering to the subject a latency reversing agent. Preferably, the latency reversing agent is a histone deacetylase inhibitor. More preferably, the latency reversing agent is romidepsin.

In a preferred embodiment of this aspect, the viral replication is controlled under 2,000 HIV-1 RNA copies/ml in plasma.

In addition to this, the inventors have surprisingly found that targeting CD33 with a specific antibody reduced virus replication and proviral integration and have thus identified CD33 as a therapeutic target in HIV infection. Thus, another aspect of the present invention relates to a CD33 inhibitor, for example, an antibody against CD33 or a fragment thereof, for use in the treatment and/or prevention of HIV-1 infection. In a preferred embodiment, said inhibitor reduces the HIV-1 viral load and/or the HIV-1 proviral levels. This aspect can also be regarded as a method for treating or prevention HIV-1 infection in a subject in need thereof, comprising administering to said subject a therapeutic amount of a pharmaceutical composition comprising a CD33 inhibitor, such as an anti-CD33 antibody or fragment thereof, or a siRNA or small molecules targeting CD33. In the same way, this aspect relates to the use of a CD33 inhibitor, such as an anti-CD33 antibody or fragment thereof, for the manufacture of a medicament for the treatment or prevention of HIV-1 infection in a subject.

As used herein, the term "comprises" also includes "consists". All embodiments of all aspects can be combined and all embodiments of all aspects are embodiments of the other aspects.

### DESCRIPTION OF THE DRAWINGS

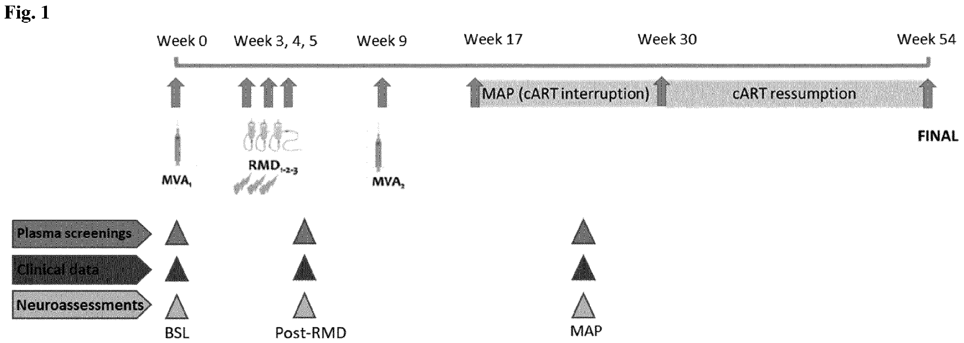

**Figure 1****.** BCN02 clinical trial scheme. Schematic representation of the BCN02 clinical trial with sampling timepoints: baseline (BSL timepoint), three RMD infusions at week 3, 4 and 5 (week 6 is post-RMD timepoint) followed by cART interruption at week 17 until viral rebound or week 30 cART resumption (MAP timepoint). Week 54 end of the BCN02 clinical trial. Analyzed timepoints labeled in arrows: baseline (BSL), post-RMD infusions (post-RMD) and monitored antiretroviral pause phase (MAP), when plasma proteomics neurological assessments were conducted.

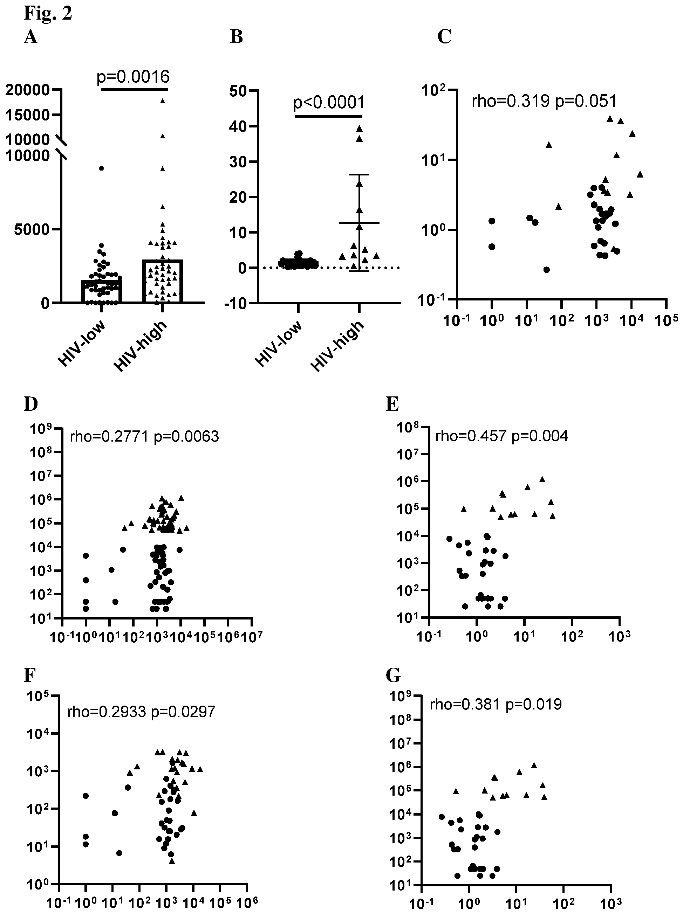

**Figure 2****.** CD33 levels in natural untreated HIV infection. Proteomic array using plasma samples from untreated HIV infected individuals with different degrees of HIV control (n=96), classed as HIV-high and HIV-low according to their viral load. **A.** Scatter plot showing the plasma CD33 protein levels (y-axis) in both groups (x-axis); HIV-low (n=49, circles) and HIV-high (n= 47, triangles). **B.** *CD33* gene expression (y-axis, relative *CD33* gene expression corrected for CD4 counts) measured in PBMCs in HIV-high (n=12, triangles) and HIV-low (n=25, circles) (x-axis) individuals. **C.** Correlation analysis between *CD33* gene expression (corrected for CD4 counts, y-axis) and CD33 protein levels in plasma considering all individuals. **D-E.** Correlation analysis between **D.** CD33 plasma protein levels and **E.** *CD33* gene expression levels (x-axis) and viral load levels (HIV RNA copies/ml, y-axis). HIV-high, n=47, triangles, and HIV-low, n=49, circles. **F-G** Correlation analysis between **F.** CD33 plasma protein levels and **G.** *CD33* gene expression levels (x-axis) and HIV proviral DNA levels in PBMC samples (HIV DNA copies/10⁶ PBMCs, y-axis). Considering all the individuals (HIV-high, n=47, triangles and HIV-low, n=49, circles. Statistical significance was set at p<0.05.

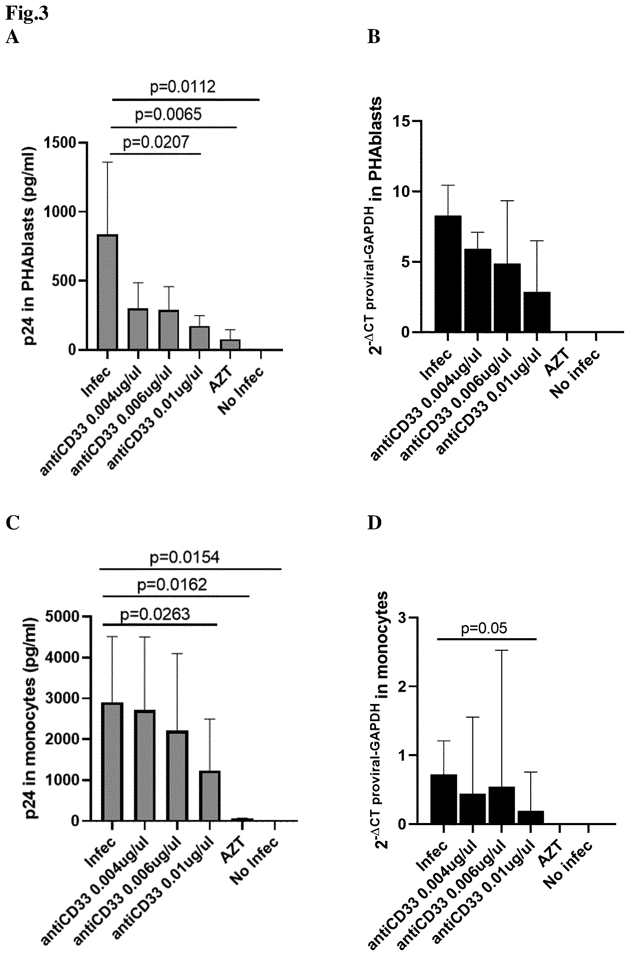

**Figure 3****.** Targeting CD33 reduces HIV replication and provirus levels. **A** and **C.** Inhibition of HIV replication in the presence of anti-CD33 mAb, tested in **A.** HIV infected PHA-stimulated T cells (six independent experiments) with HIV_{NL4-3} strain; **B.** HIV infected monocyte-derived macrophages (five independent experiments) infected with HIV_{BaL} strain. Experimental conditions are shown on the x-axis and quantification of absolute p24 supernatant (pg/ml). **B** and **D** Proviral quantification in cells from the same T cell and MDMs experiments as in A and C. Experimental conditions are shown on the x-axis and quantification of proviral is shown on the y-axis. For HIV infected T cells and MDM, ANOVA test was used to analyze differences between conditions. For all comparisons, p<0.05 was considered statistically significant. The plots show the median of all experiments for each condition.

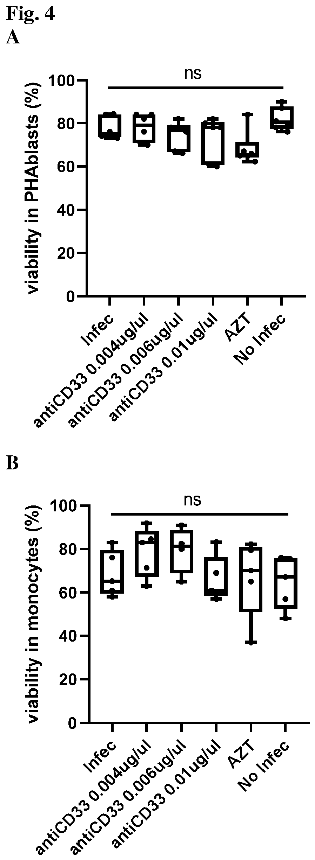

**Figure 4****.** Assessment of cellular toxicity of anti-CD33 mAb. Cell viability in the presence of increasing concentration of the anti-CD33 mAb, tested in **A** HIV infected PHA-stimulated T cells (six independent experiments) with HIV_{NL4-3} strain; and in **B** monocyte-derived macrophages (five independent experiments) infected with HIV_{BAL} strain. Experimental conditions are shown on the x-axis; quantification of viability (% live cells) is shown on the y-axis.

### EXAMPLES

The following examples illustrate the present invention:
**Plasma Proteomes impacted upon RMD administration in BCN02 clinical trial.**

Focused inflammatory and neurological-tailored proteomes analysis using Proximity Extension Assay (PEA) were defined in plasmas samples from 11 BCN02 participants at baseline (BSL, week 0), 1 week after 3 infusions of romidepsin (Post-RMD, week 6) and monitored antiretroviral pause (MAP) timepoints (Figure 1), to identify soluble markers modulated by therapeutic vaccination and the latency reversal agent romidepsin. Principal Component Analysis (PCA) was based on 276 soluble factors involved in inflammation and neurological processes and which were assessed across 3 timepoints of the clinical trial and showed that the most marked changes are observed after RMD treatment and were intensified during MAP. When compared to baseline, the administration of RMD modulated the plasma levels of 49 proteins, while 76 molecules were modulated during MAP levels. Proteome profiling analysis indicated that of 20 proteins differentially detected only between baseline and RMD administration timepoints, 17 molecules showed an increase in relative protein levels, most of these proteins were related to "cytokine release by the interaction with viral proteins" such as CCL25, CXCL5, IL20RA and IL15 and consequently "innate and cellular adaptive immune response markers" like CD8A and CD38. On the other hand, 3 proteins showed decreased relative levels after RMD administration, including CXCL10, CCL23 and GDNF. When comparing baseline versus MAP timepoints, 47 molecules showed different relative protein levels, with 37 having increased relative protein levels, most of these proteins were associated with "cell growth", "proliferation" or "cell structure" such as TBCB, PFDN2, 4E-BP1, NPM1, FGF23, CD63, among others. The 10 proteins that decreased their relative levels over-time were related to "response to cytokine" category like IL20, IL22RA1, LIF, GBP2, SCGB1A1 and NEP and "regulation of epithelial cell differentiation" such as GDNF, LIF and IL20. In addition, the 29 common proteins between baseline versus post-romidepsin and baseline versus MAP comparisons, were progressively and significantly increased (24 proteins) or decreased (5 proteins) over-time. Most of the increased 24 proteins were involved in "neuronal and axonal functions" like NEFL, MANF and CLSTN1, while the other 5 molecules that decreased (MCP3, IL5, TNF, CEACAM3 and TDGF1) showed an opposite profile.

To identify potential drivers of the observed changes in protein levels from baseline to post-RMD and/or MAP timepoints, a correlation analysis was performed between relative protein levels and either virological (plasma viral load (VL) and proviral levels) or neurological parameters (NPZ6). This analysis showed that after RMD infusions 34% plasma proteins correlated positively with pVL, whereas during MAP timepoint this percentage increased up to 72% of the molecules (p=0.0001, chi squared test). Oppositely to pVL associations, at post-RMD timepoint, 72% of plasma proteins were positively associated with proviral levels, while during MAP phase these associations decreased to 31% (p=0.0001, chi squared test). In general, this data show that while the proteome at post-RMD is more directly driven by viral reservoir, during MAP the significant proteomes are more associated with pVL, especially proteins directly involved in "interferon signaling pathway" such as IL15, CXCL10, IFI30, TNF, IL3, IL12, IL20. Interestingly, correlation analysis with neurological parameters (NPZ6) showed that 71% of the molecules correlated positively with NPZ6 neurocognitive test after RMD infusion. During MAP, this proportion rose to 85% (p=0.0083, chi squared test), evidencing the higher number of proteins associated with neurological evaluations over the intervention and including proteins such as MANF, GDNF, NEFL GPNMB.

### Plasma proteomes at baseline already differentiate MAP-Controllers and MAP-Non-Controller.

Having observed plasma proteomes being associated with viral loads and proviral levels, we assessed whether baseline or longitudinally changed proteome signatures could differentiate individuals who would go on to control viral replication during MAP. For that, we compared the plasma proteomes of MAP-NC individuals (>2000 HIV RNA copies/ml and thus restarting ART) and MAP-C (>32 weeks <2000 HIV RNA copies/ml) in all the timepoints (baseline, post-RMD and MAP). PCA plots showed that proteome profiles at all timepoints, including baseline, segregated the two groups. Specifically, the comparative analysis between MAP-C and MAP-NC by timepoints showed that differences in the proteome of both groups were observed at baseline (18 proteins), post-RMD (16 molecules) and, with broader profiles, during MAP (33 molecules).

Gene Ontology (GO) classification was performed on all these differently expressed proteins. In addition to metabolic and hormonal ontologies, the analysis at baseline indicated a differential representation between MAP-C and MAP-NC in "T cell differentiation", "lymphocyte immunity", "interferon-gamma production" and "myeloid cell development" ontologies. After RMD administration but before vaccination, the major gene ontologies differentially represented between MAP-C and MAP-NC fell into several "interferon pathways" and in "negative regulation of IL5 and IL13 production" ontologies. "Innate cellular (Mast cells, Neutrophils and Monocytes)", "T cell immunity" categories and the involvement of "NF-KB and MAPK cascades" were the major ontologies differentially represented between both groups at the MAP timepoint.

**CD33 (Siglec-3) is the unique discriminative plasma factor between MAP-C and MAP-NC across the timelines of the BCN02 clinical trial.**

When analyzing the proteome profiles discriminating MAP-C and MAP-NC, we surprisingly found that CD33 (Siglec-3) was the unique protein that was consistently differentially detected between the two groups across all timepoints tested. Moreover, the plasma levels of CD33 were modulated during the intervention (BSL vs post-RMD p=0.001 and BSL vs MAP p=0.002, Wilcoxon test) and were significantly elevated in MAP-NC compared with MAP-C in all timepoints (BSL: p=0.024, post-RMD: p=0.049 and MAP: p=0.033, Mann-Withney test). In addition, CD33 protein plasma levels positively correlated with proviral copy numbers at baseline and after RMD administration (BSL rho=0.646 p=0.037, post-RMD rho=0.647 p=0.035, Spearman rank test). A positive association was also observed at MAP timepoint (MAP rho=0.9 but with p=0.083). Association with pVL at MAP phase indicated that CD33 protein levels also positively correlated with pVL (rho=0.782 p=0.011, Spearman rank test).

We further investigated whether cells in the peripheral blood are the major source of CD33 in plasma. We assessed CD33 gene expression levels in PBMC. While MAP-NC and MAP-C expressed same levels of CD33 at baseline, MAP-C showed a trend towards reduced CD33 expression in comparison to MAP-NC, along with a positive correlation between CD33 expression and plasma viral load levels (rho=0.655, p=0.034), reflecting the observed correlation between CD33 protein plasma levels and viral load (rho=0.782, p=0.011).

To gain further insight in the mechanisms by which CD33 could influence viral control, we performed a correlation analysis between CD33 and other proteins included in the inflammatory, neurology or neuro-exploratory cytokine panels that significantly changed during the intervention. After RMD administration, CD33 protein levels were strongly associated with 17 proteins, most significantly with ADAM15, GGT5 and NEFL (ADAM15 rho=0.806 p=0.0027, GGT5 rho=0.7413 p=0.009 and NEFL rho=0.7064 p=0.0151, Spearman rank test). These association were not only maintained but statistically even more pronounced at the MAP timepoint (ADAM15 rho=0.9696 p<0.0001, GGT5 rho=0.9195 p=0.0002 and NEFL rho=0.9223 p=0.0001, Spearman rank test). Interestingly, the NEFL protein, a well-established marker for neurological damage in several diseases (10, 11), was also detected at increased levels upon RMD administration and was accentuated in MAP phase, but did not reach significant difference between groups, even in baseline timepoint where MAP-NC tended to have higher levels of this brain injury biomarker (p=0.0095 in BSL vs Post-RMD and 0.0239 in Post-RMD vs MAP).

Overall, these surprising data indicate that CD33 protein levels differ between MAP-C and MAP-NC, even before any intervention and that with the administration of RMD this difference is intensified and related with viral parameters. Interestingly, we identified a surprising direct relationship of CD33 with other plasmatic molecules, especially also with the neurological damage marker, NEFL.

### CD33 (Siglec-3) validation in chronic untreated HIV infected cohort.

To validate the relationship between CD33 plasma levels and virus control, we tested samples from a cohort of untreated chronically HIV-1 infected individuals with different levels of virus control by determining plasma protein and gene expression levels of CD33 and compared them to virus control in natural HIV infection. This independent untreated HIV infection cohort included HIV infected individuals with high (as "HIV-high", n= 47, pVL >50,000 HIV RNA copies/ml) or low ("HIV-low", n= 49, pVL < 10,000 HIV RNA copies/ml) plasma viral loads. Significantly higher CD33 plasma protein levels were detected in HIV-high compared with HIV-low individuals (Figure 2A, p=0.0016, Mann-Whitney test). Similarly, CD33 gene expression in PBMCs, from the same cohort, showed higher *CD33* expression levels in HIV-high individuals compared with HIV-low (Figure 2B, p<0.0001, Mann-Withney test). CD33 proteomic and gene expression levels at PBMCs correlated positively (Figure 2C, rho=0.319 p=0.051, Spearman rank test) indicating that PBMCs are one of the major sources of CD33 protein levels in the plasma. As observed in the MAP phase of the BCN02 study, CD33 plasma and gene expression levels also correlated with pVL (Figure 2D-E and H, CD33 protein levels vs pVL rho=0.2771 p=0.0063, and CD33 gene expression levels vs pVL rho=0.457 p=0.004, Spearman rank test) and HIV proviral copy numbers (Figure 2F-G, CD33 protein levels vs proviral rho=0.2933 p=0.0297, and *CD33* gene expression levels vs proviral rho=0.381 and p=0.019, Spearman rank test).

These data further indicate that CD33 (Siglec-3) is closely related with in vivo viral replication and serves as marker of natural infection control and as a predictor of the outcome after treatment interruption and/or therapeutic interventions.

### In vitro CD33 targeting reduces HIV replication and virus reactivation

We next investigated more directly the involvement of CD33 in HIV replication and viral reservoir. Specifically, we tested whether targeting of CD33 *in vitro* on T cells and monocyte-derived macrophages (MDM) impacted in HIV infection. Indeed, adding an anti-CD33 monoclonal antibody (mAb) to HIV_{NL4-3}-infected PHA-stimulated T cell cells reduced HIV replication in a dose-dependent manner (Figure 3A) without affecting the cell viability (Figure 4A). In parallel, the amount of the integrated proviral DNA was also decreased in the presence of anti-CD33. The same was observed when HIV_{BaL}-infected MDMs were cultured in presence of anti-CD33, with HIV replication being reduced in a dose-dependent manner (Figure 5B p=0.0263 and S4B, ANOVA test). The reduction in proviral copy numbers in monocytes reached statistical significance as well (Figure 5C). Together, these data show that CD33 targeting reduces HIV replication, indicating that CD33 at some point of the HIV viral infection is required for effective viral infection in peripheral blood cells.

### Materials and Methods

**Patients and samples:** The BCN02 clinical trial (NCT02616874) was a phase I, open-label, single-arm, multicenter study conducted in Barcelona, Spain (2). Fifteen participants, who were roll-over from the previous BCN01 trial, were immunized with MVA.HIVconsv followed by three weekly-doses of romidepsin (5 mg/m²) and a second MVA.HIVconsv vaccination before undergoing a monitored antiretroviral pause (MAP) for a maximum of 32 weeks (Figure 1). During MAP, the participants restarted cART treatment if they reached the threshold of 2000 HIV RNA copies/ml or completed the 32 weeks with controlled viral replication. Available plasma samples from 11 participants were used for the longitudinal proteomic profiling analysis, including participants that did not control virus during MAP (8 MAP-NC) and those that completed the 32 weeks of MAP (3 MAP-C). Chronic untreated HIV infected individuals (n=96), enrolled at the IMPACTA clinics (Peru) and Hospital Germans Trias i Pujol (Spain), were classified according to their degree of control of viral replication; HIV-high n=47, >50,000 HIV RNA copies/ml and HIV-low n=49, < 10,000 HIV RNA copies /ml. Additionally, CD4 counts measured in HIV-high ranged between 11-729 cells/mm³ and in HIV-low from 289-1,343 cells/mm³. Available plasma and dry-pellet PBMC were stored until use. Blood samples from HIV uninfected donors from the Banc de Sang i Teixits in Barcelona for in vitro studies were used.

**Plasma Proteomic Profiling:** BCN02 plasma samples were used for evaluation of inflammation, neurology and neuro-exploratory cytokine panels applying proximity extension assays by Olink^{®} (https://www.olink.com/data-you-can-trust/technology/) Relative expression levels were expressed as normalized protein expression (NPX). For validation of relative CD33 plasma levels in HIV untreated chronic HIV infected individuals proteomic analysis were performed using customized arrays as previously described (7). Protein-Protein Interaction Networks and Functional Enrichment Analyses were done using free access databases (http://geneontology.org/ and (https://string-db.org/).

### GEO access of BCN02 transcriptomics

Gene expression of CD33 was obtained from previously published work by Oriol-Tordera et al (6). The dataset is available in Gene expression Omnibus (GEO), under the accession number GSE184653. The specific values for CD33 were obtained from that dataset after log2 transformation of the TMM normalized counts.

**Evaluation of CNS functioning:** Neuropsychological evaluation covered 6 cognitive domains to provide a global composite score (NPZ-6) which was recorded as recently published (8). In brief, NPZ6 included: Digit Test of the Wechsler Adult Intelligence Scale (WAIS-IV); the Trail Making Test (TMT-A) and the Symbol Digit Modalities Test (SDMT); Grooved Pegboard Test (GPT); California Verbal Learning Test (CVLT-II); the Initial Letter "p" and the Animals Test; and the Trail Making Test (TMT-B) and the Tower of London Test (TOL). The Vocabulary Test of the WAIS-IV was used to estimate premorbid intelligence.

**Determination of integrated HIV proviral DNA:** HIV proviral DNA was quantified in PBMCs samples of untreated, chronically HIV-1 infected individuals and in isolated CD4⁺ T cells of BCN02 clinical trial participants by droplet digital PCR (ddPCR) in duplicates as previously reported (9). Briefly, two different primer/probe sets annealing to the 5' long terminal repeat and Gag regions, respectively, were used to circumvent sequence mismatch in the patient proviruses, and the *RPP30* housekeeping gene was quantified in parallel to normalize sample input. Raw ddPCR data were analyzed using the QX100^{™} droplet reader and QuantaSoft v.1.6 software (Bio-Rad).

**Real-time PCR CD33 gene:** RNA samples from available PBMC dry-pellets from chronic untreated HIV infected cohort were retrotranscribed and TaqMan gene expression assay (Applied Biosystems) was used for *CD33* (hs01076281_m1) detection. *TBP gene* (Hs99999910_m1) *was used as the housekeeping gene.* Gene amplification was performed in Applied Biosystems 7500 Fast Real-Time PCR System thermocycler, and the relative expression quantification was calculated as 2^{-Δ*CT*} (where *C_{T}* is the median threshold cycle from 3 replicates).

**HIV infection of PHA-blasts and monocyte-derived macrophages:** Isolated PBMCs from HIV uninfected donors were stimulated with PHA (5µg/ml) and IL-2 (10U/ml). After 3 days, PHA-blasts were infected with the HIV_{NL4-3} strain (Multiplicty of Infection (MOI): 0.01). For monocyte-derived macrophages (MDMs), PBMCs were depleted using the EasySep^{™} Human Monocyte Enrichment Kit (Stem Cell). Monocytes were then incubated with macrophage colony-stimulating factor (M-CSF) at 1µg/ml for 4 days before infection with the HIV_{BaL} strain (MOI: 0.01). HIV infection in PHA-blasts and MDMs was evaluated under different conditions: zidovudine (AZT, 200µg/ml) as viral inhibition control and anti-CD33 titration (Abcam; Rabbit monoclonal [EPR4423] to CD33) was used at final concentrations of 0.004ug/ul, 0.006ug/ul and 0.01ug/ul. After 3 (PHA stimulated T cell blast) and 4 days (MDM), p24 in supernatant was quantified by ELISA (INNOTEST HIV p24 Antigen mAb). HIV proviral quantification was performed on total DNA isolated from cultured cells using the RNA/DNA purification Micro kit (Norgen Biotek corp.) as recommended by the manufacturer. Quantification of HIV proviral was determined by a quantitative PCR assay, using TaqMan Universal Master Mix II (Applied Biosystems) in a 7500 real-time PCR system (Applied Biosystems). We used the following set of primers and probe, forward primer, 5'-GACGCAGGACTCGGCTTG-3'; reverse primer, 5'-ACTGACGCTCTCGCACCC-3'; probe, 5'-fluorescein amidite (FAM)-TTTGGCGTACTCACCAGTCGCCG-6-carboxytetramethylrhodamine (TAMRA)-3'. *GAPDH* gene (Thermo Fisher Assay ID Hs02786624_g1) was used as the housekeeping gene. Gene amplification was performed in an Applied Biosystems 7500 Fast Real-Time PCR System thermocycler, and the relative expression quantification was calculated as 2-Δ*CT* (where *CT* is the median threshold cycle from 2 replicates).

**Statistical analysis:** Univariate and multiple comparisons statistical analyses were performed using GraphPad Prism, version 8. For comparisons between patient groups, the Mann-Whitney and Wilcoxon signed rank tests were applied and for multiple group comparisons one-way ANOVA test with False Discovery Rate (FDR) calculation using Benjamini and Hochberg method were used. The Spearman and Pearson test were applied for the correlation analysis with non-parametric and parametric data, respectively. For all analyses, p-values <0.05 were considered statistically significant. Principal Component Analysis (PCAs), Heatmaps, Venn diagrams and correlograms were perform with RStudio version 1.2.5042

**Ethics Approval:** The study was approved by the Comité Ètic d'Investigaci6 Clínica of Hospital Germans Trias i Pujol (CEIC: EO-12-042 and PI-18-183) and all participants provided their written informed consent. All the research involving human research participants was performed in accordance with the Declaration of Helsinki.

### References

1. Cillo AR, Mellors JW. Curr Opin Virol. 2016;18:14-9.
2. Mothe B, et al. Front Immunol. 2020;11(May):1-15.
3. Rosás-Umbert M, et al. Front Immunol. 2020;11(March):1-11.
4. Wei DG, et al. PLoS Pathog. 2014;10(4).
5. Borgognone A, et al. bioRxiv. 2021;2021.10.03.462590.
6. Oriol-Tordera B, et al. eBioMedicine. 2022;78:1-19.
7. Oriol-Tordera B, et al. HHS Public Access. 2021;205(12):3348-57.
8. Muñoz-Moreno JA, et al. AIDS. 2022 Mar 1;36(3):363-372..
9. Martínez-Bonet M, et al. Clin Infect Dis. 2015;61(7):1169-78.
10. Liu Y, et al. Front Neurosci. 2022;16(September):1-10.
11. Ning L, Wang B. PLoS One. 2022 Sep 14, 17(9):e0274565.
12. Gonzalez-Gil A, Schnaar RL. Cells. 2021; 10(5): 1-21.
13. Zhao L. Gerontology. 2019;65(4):323-31.

## Claims

1. Use of CD33 as a biomarker of the response to a T-cell vaccine in a HIV-1 infected subject.

2. The use according to claim 1, wherein the T-cell vaccine is MVA.HIVconsv vaccine or a HTI immunogen-based vaccine.

3. The use according to any one of claims 1 or 2, wherein the levels of CD33 mRNA or CD33 protein are used as biomarker, preferably the levels of CD33 protein are used as biomarker, more preferably the levels of soluble CD33 protein are used as biomarker.

4. The use according to any one of claims 1 to 3, wherein said use is performed in a sample from said subject, wherein the sample is selected from blood, plasma, serum, peripheral blood mononuclear cells, urine, cerebrospinal fluid and combinations thereof, preferably the sample is selected from blood, plasma, serum, peripheral blood mononuclear cells and combinations thereof, more preferably the sample is plasma.

5. The use according to any one of claims 1 to 4, wherein the subject is under therapy to control viral load and/or replication, preferably combined antiretroviral therapy or anti CD-33 therapy.

6. The use according to any one of claims 1 to 5, wherein a positive response to the vaccine is delaying the time until viral rebound and/or controlling the plasma virus under 2,000, preferably under 50 HIV-1 RNA copies/ml, upon the interruption of the therapy, preferably combined antiretroviral therapy.

7. The use according to any one of claims 1 to 4, wherein the biomarker levels are analyzed:
a. before administering the vaccine,
b. after administering the vaccine,
c. before interrupting the therapy to control viral load and/or replication,
d. After interrupting the therapy to control viral load and/or replication, or
e. any combination of (a) and (c), or (b) and (c) or (b) and (d).

8. Use of CD33 as a biomarker of viral load, viral replication and/or viral reservoir in a HIV-1 infected subject.

9. The use according to claim 8, wherein the levels of CD33 mRNA or CD33 protein are used as biomarker, preferably the CD33 plasma levels or the CD33 mRNA levels in peripheral blood mononuclear cells.

10. A method for the prognosis of the response of a HIV-1 infected subject to a T-cell vaccine comprising:
a. analyzing the CD33 levels in a sample from said subject;
b. administering the vaccine to the subject; and
c₁. prognosing that the viral replication will be controlled, if the results of step (a) correspond to the low levels found in the group of subjects that respond to the vaccine; or
c₂. prognosing that the viral replication will not be controlled, if the results of step (a) correspond to the high levels found in the group of subjects that do not respond to the vaccine.

11. The method according to claim 10, wherein the subject is under a therapy to reduce viral load or viral replication, preferably the subject is under combined antiretroviral therapy or under anti-CD33 therapy.

12. The method according to any one of claims 10 or 11, wherein the T-cell vaccine is MVA.HIVconsv vaccine or a HTI immunogen based vaccine.

13. The method according to any one of claims 10 to 12, wherein step (b) further comprises administering to the subject a latency reversing agent, preferably wherein the latency reversing agent is a histone deacetylase inhibitor, more preferably wherein the latency reversing agent is romidepsin.

14. The method according to any one of claims 10 to 13, wherein the viral replication is controlled under 2,000, preferably under 50 HIV-1 RNA copies/ml in plasma.

15. A CD33 inhibitor for use in the treatment and/or prevention of HIV-1 infection, preferably wherein said inhibitor is an antibody or fragment thereof, more preferably wherein said inhibitor reduces the HIV-1 viral load and/or the HIV-1 proviral levels.
